# EUROPEAN PATENT SPECIFICATION

(11) **EP 0 869 960 B1**
(45) Date of publication and mention of the grant of the patent: **03.07.2002**
(21) Application number: 96906103.5
(22) Date of filing: 25.03.1996
(51) Int. Cl.: C07D 499/00, C12P 37/04

(54) **PROCESS FOR THE RECOVERY OF AMPICILLIN**
VERFAHREN ZUR WINNUNG VON AMPICILLIN
PROCEDE DE RECUPERATION D'AMPICILLINE

(30) Priority: 31.03.1995 BE 9500291
(43) Date of publication of application: 14.10.1998
(73) Proprietor: DSM N.V., 6411 TE Heerlen (NL)
(72) Inventor: BOESTEN, Wilhelmus, Hubertus, Joseph, NL-6132 BJ Sittard (NL); MOODY, Harold, Monro, NL-6211 BZ Maastricht (NL); ROOS, Eric, Cornelis, NL-6211 BX Maastricht (NL)
(86) International application number: NL9600128
(87) International publication number: WO9630376

(56) References cited:
- WO-A-95/03420
- DE-A- 2 611 286
- FR-A- 2 188 608
- GB-A- 994 402
- US-A- 4 354 971

## Description

The invention relates to a process for the recovery of ampicillin from a mixture containing ampicillin and 6-aminopenicillic acid (6-APA).

In the preparation of ampicillin, with 6-APA being acylated with a D-phenylglycine derivative, the recovery of the ampicillin and working up of the reaction mixture are difficult in general.

A process for isolating the ampicillin pure from a mixture containing ampicillin and minor quantities of 6-APA is described in JP-A-47030687. According to the process described in this Japanese publication, an acid aqueous mixture containing 6-APA and ampicillin is subjected to an extraction with butanol or isoamylalcohol, after which the pH is raised to a value between 6 and 7 and the product is recovered by complete boiling down and freeze-drying. The drawback of this method is that organic solvents that are alien to the process have to be added. In addition, complete boiling down and freeze-drying is not industrially practicable. Moreover, the process involves formation of salts that are included in the freeze-dried product. French patent 2.188.608 discloses a process for the preparation of penicillins. In Example 1 and 2, the preparation of ampicillin is described. In the French patent it is stated, that after the preparation process the reaction mixture is worked up " in the usual manner". A specific process for the recovery of Ampicillin, however, is not disclosed in the examples or in the description. There is no indication which measures are involved in "the usual manner".

The object of the invention is to provide a simple, industrially practicable process allowing ampicillin to be recovered in pure form without making use of said organic solvents that are alien to the process.

This is achieved according to the invention in that a mixture containing ampicillin and 6-APA and having a pH higher than 7, which, apart from any solid ampicillin that is present, is homogeneous at a pH between 7 and 8.5, is subjected to a pH lowering to a pH lower than 8.2, and that the solid substance present is recovered.

The applicant has found that by lowering the pH of the mixture to a value lower than 8.2, for instance between 5 and 8, in particular between 5.5 and 7.8, depending on the composition of the reaction mixture, it is possible to cause ampicillin to be crystallized out with a purity of more than 90 mass%, in particular more than 98 mass%, even if a large amount of 6-APA is present in the mixture, after which it can be recovered. Besides ampicillin the reaction mixture often contains other valuable components, such as for instance the 6-APA. In order to get a commercially attractive process it is consequently important also to minimize the 6-APA and ampicillin losses. It has also been found that if subsequently the pH is lowered further to a value of less than 7, in particular between 1.5 and 6, 6-APA and the remaining ampicillin crystallize out virtually completely, after which this mixture of 6-APA and ampicillin can be recovered, for instance by filtration. The resulting mixture of 6-APA and ampicillin can optionally be re-used, so that a process is obtained which yields pure ampicillin without significant losses of 6-APA and ampicillin.

The process according to the invention is in particular suitable to be applied in the working up of the reaction mixture which is obtained after the enzymatic acylation reaction in which 6-APA is acylated with D-phenylglycine amide (PGA) or esters of D-phenylglycine. Thus, the process according to the invention can for instance be applied to a starting mixture that is obtained by successively filtering, with isolation of (immobilized) enzyme, the reaction mixture of an enzymatic acylation reaction carried out at a relatively high pH, for instance a pH between 7.5 and 10, in particular between 8 and 10, lowering the pH to a value between 7 and 9. Depending on the amount of D-phenylglycine (PG) formed during the acylation reaction, if desired, it is possible to remove first at a higher pH-value between 7 and 9 - which is chosen dependent on the mixture such that already PG has been crystallized out and ampicilline has not yet - the eventually formed solid substance, which mostly will consist mainly of PG.

In another embodiment the starting mixture used is the mixture obtained after an enzymatic acylation reaction that ends at a relatively low pH, for instance a pH between 7 and 8.8, preferably between 7.5 and 8.8, and after isolation of the solid substance which mainly contains the immobilized enzyme and D-phenylglycine.

In the starting mixture containing 6-APA and ampicillin, a significant quantity of 6-APA may be present. The quantity of 6-APA is mostly less than 75 mol% relative to the total amount of 6-APA plus ampicillin, preferably 2-60%, in particular 5-50%.

The pH may be lowered in several ways in the framework of the invention, for instance chemically by adding an acid, for instance a mineral acid, in particular sulphuric acid, hydrochloric acid or nitric acid. Another possibility is for instance, if PGA has been used as acylation agent in the acylation reaction or if an ester of PG has been used and the pH has been kept constant by means of titration with ammonia during the acylation reaction, to lower the pH through physical removal of ammonia. Suitable physical removal methods are for instance stripping with steam or an inert gas; (steam) distillation at reduced pressure, in particular thin-film evaporation; evaporation in a spray tower; gas membrane separation or electrodialysis.

The optimum pH at which ampicillin is recovered depends on the composition of the mixture and is chosen such that optimum separation of 6-APA and ampicillin is achieved. In practice the optimum pH is a compromise between on the one hand high purity of the ampicillin recovered, which is achieved if the pH at which the ampicillin is recovered is relatively high, so that the ampicillin is still partly in solution and the 6-APA still completely in solution, and on the other hand a high yield, which is achieved if the pH at which the ampicillin is recovered is relatively low so that the ampicillin has been precipitated virtually completely, while at the same time part of the 6-APA has also been precipitated. For the person skilled in the art it is easy to determine the optimum pH in a given situation.

The temperature at which the working up is performed is mostly lower than 35°C, preferably between 0 and 30°C, in particular between 10 and 30°C.

The process according to the invention for recovery of pure ampicillin in combination with recirculation of the mixture of 6-APA and ampicillin obtained after further pH lowering, applied to the mixture obtained after enzymatic acylation of 6-APA with PGA enables an overall high selectivity towards 6-APA to be achieved, in particular higher than 80%. The remaining filtrate, which mainly contains minor residual amounts of PGA, may optionally be worked up further, for instance by bringing it to a pH higher than 8, in particular between 8.5 and 10. If desired, it is possible to apply further concentration and cooling to a temperature lower than 10°C, for instance between 0 and 8°C. In this way a process is obtained with which both 6-APA and PGA can be applied with a high efficiency. In the enzymatic acylation reaction, PGA or esters of PGA for instance may be used as acylation agent.

In principle any enzyme can be used that is suitable as catalyst in the coupling reaction. Such enzymes are for instance the enzymes that are known under the general designations 'penicillin amidase' and 'penicillin acylase'. Examples of suitable enzymes are enzymes derived from Acetobacter, Aeromonas, Alcaligenes, Alcaligenes, Aphanocladium, Bacillus sp., Cephalosporium, Escherichia, Flavobacterium, Kluyvera, Mycoplana, Protaminobacter, Pseudomonas and Xanthomonas, in particular Acetobaxter pasteurianum, Bacillus megaterium, Escherichia coli and Xanthomonas citrii.

Preferably an immobilized enzyme is used, since the enzyme can be easily isolated and re-used then. Immobilized enzymes are known as such and are commercially available. Example of suitable enzymes are the Escherichia coli enzyme from Boehringer Mannheim GmbH, which is commercially available under the name 'Enzygel®', the immobilized Penicillin-G acylase from Recordati, the immobilized Penicillin-G acylase from Pharma Biotechnology Hannover, and an Escherichia coli penicilline acylase isolated as described in WO-A-92/12782 and immobilised as described in EP-A-222462.

The enzymatic acylation reaction is mostly carried out at a temperature lower than 35°C, preferably between 0 and 28°C. The pH at which the enzymatic acylation reaction is carried out is mostly between 5.5 and 10, preferably between 6 and 9.

In practice the enzymatic acylation reaction and the working up of the reaction mixture are mostly carried out in water. Optionally, the reaction mixture may also contain an organic solvent or a mixture of organic solvents, preferably less than 30 vol.%. Examples of organic solvents that can be used are alcohols with 1-7 carbon atoms, for instance a monoalcohol, in particular methanol or ethanol; a diol, in particular ethylene glycol or a triol, in particular glycerol.

In the framework of the present invention the various components may be present in the reaction mixture in the free form or as salts. The pH values mentioned are in all cases the pH values measured at room temperature.

The invention will be further elucidated by means of the following examples, without however being restricted thereto.

### Abbreviations:

AMPI = ampicillin
AMPI.3H₂O = ampicillin trihydrate
6-APA = 6-aminopenicillic acid
PGA = D-phenylglycine amide
PG = D-phenylglycine

### Example I

Enzymatic coupling of 200 mM of PGA and 200 mM of 6-APA at 5°C, followed by working up.

A mixture of 43.9 g of 6-APA and 30.6 g of PGA was suspended in 877 ml of water and cooled to 5°C. The resulting suspension was added to 100 g of 'wet' immobilized Pen-G acylase from Recordati (Milan). This enzyme is commercially available in a mixture of water and glycerol ('wet enzyme'); before use it was washed three times with 100 ml of water.

After 2 hours the pH had risen to 8.16. By means of concentrated aqueous NH₃ the pH was brought to 8.6 and after 5 minutes the reaction mixture was filtered through a G-3 glass filter; the residue was washed with 100 ml of water (5°C). This residue was a mixture of enzyme and PG formed during the reaction.

The combined filtrate was acidified to pH 6.8 at 5°C in 10 minutes by means of concentrated H₂SO₄. After 30 minutes, filtering, washing with 3 x 30 ml of water and drying were performed, which yielded 30.6 g of AMPI.3H₂O with a chemical purity (on a water-free basis) of > 98%.

The mother liquor of the AMPI crystallization was again acidified to pH = 4.6 with concentrated H₂SO₄ at 5°C. After stirring for 1 hour, filtering and washing with 2 x 20 ml of water were performed, which yielded 13.0 g of solid substance, calculated on a water-free basis. This solid substance contained a mixture of APA and AMPI in a molar ratio of 94:6.

### Example II

Enzymatic coupling of 200 mM of PGA and 200 mM of 6-APA at 5°C, followed by working up.

A mixture of 43.9 g of 6-APA and 30.6 g of PGA was suspended in 877 ml of water and cooled to 5°C. The resulting suspension was added to 100 g of 'wet' immobilized Pen-G acylase from Recordati (Milan). This enzyme is commercially available in a mixture of water and glycerol ('wet enzyme'); before use it was washed three times with 100 ml of water.

After 2 hours the pH had risen to 8.16. By means of concentrated aqueous NH₃ the pH was brought to 8.6 and after 5 minutes the reaction mixture was filtered through a G-3 glass filter; the residue was washed with 100 ml of water (5°C). This residue was a mixture of enzyme and PG formed during the reaction.

The combined filtrate was worked up as follows: by means of a rotary film evaporator 510 ml of liquid was distilled off at 20°C (in about 45-60 minutes), after which 510 ml of water was added. This procedure was repeated one time. The resulting suspension was filtered through a G-3 glass filter and washed with 2 x 30 ml of water (5°C) and dried, which yielded 22.2 g of AMPI.3H₂O of a chemical purity (on a water-free basis) of > 99%.

## Claims

1. Process for the recovery of ampicillin from a mixture containing ampicillin and 6-aminopenicillic acid (6-APA), **characterized in that** a mixture which contains ampicillin and 6-APA, with a pH higher than 7, which apart from any solid ampicillin being present is homogeneous at a pH between 7 and 8.5, is subjected to a pH lowering till a pH lower than 8.2 is reached, and the solid substance present is recovered.

2. Process according to claim 1, **characterized in that** the pH is lowered to a value between 5.5 and 7.8.

3. Process according to claim 1 or 2, **characterized in that** the starting mixture contains 2-60 mol% of 6-APA, calculated relative to the total amount of 6-APA and ampicillin.

4. Process according to any one of claims 1-3, **characterized in that** the remaining liquid phase is subjected to a pH lowering to a pH lower than 7 and the solid substance present is recovered.

5. Process according to claim 4, **characterized in that** the remaining liquid phase is subjected to a pH lowering to a pH between 1.5 and 6.

6. Process according to any one of claims 1-5, wherein the mixture of ampicillin and 6-APA is prepared from the reaction mixture of an enzymatic acylation reaction carried out at a pH between 7.5 and 10, through lowering the pH to a value between 7 and 9 and isolating the solid substance.

7. Process according to claim 6, wherein first the solid substance present is isolated from the reaction mixture obtained after the acylation reaction.

8. Process according to any one of claims 1-5, wherein the mixture of ampicillin and 6-APA is prepared from the reaction mixture of an enzymatic acylation reaction at a pH between 7 and 8.8, with isolation of the solid substance present.

## Patentansprüche

1. Verfahren zur Rückgewinnung von Ampicillin aus einer Ampicillin und 6-Aminopenicillinsäure (6-APA) enthaltenden Mischung, **dadurch gekennzeichnet, dass** eine Mischung, die Ampicillin und 6-APA enthält, mit einem pH-Wert von mehr als 7, die abgesehen von jeglichem vorhandenen festen Ampicillin, bei einem pH-Wert zwischen 7 und 8,5 homogen ist, einer pH-Wert-Absenkung unterzogen wird, bis ein pH-Wert von unter 8,2 erreicht ist und die vorhandene feste Substanz rückgewonnen wird.

2. Verfahren nach Anspruch 1, **dadurch gekennzeichnet, dass** der pH-Wert auf einen Wert zwischen 5,5 und 7,8 abgesenkt wird.

3. Verfahren nach Anspruch 1 oder 2, **dadurch gekennzeichnet, dass** die Ausgangsmischung 2-60 Mol-% 6-APA, in Bezug auf die Gesamtmenge von 6-APA und Ampicillin berechnet, enthält.

4. Verfahren nach einem der Ansprüche 1-3, **dadurch gekennzeichnet, dass** die übrige flüssige Phase einer pH-Wert-Absenkung auf einen pH-Wert von weniger als 7 unterzogen wird und die vorhandene feste Substanz gewonnen wird.

5. Verfahren nach Anspruch 4, **dadurch gekennzeichnet, dass** die übrige flüssige Phase einer pH-Wert-Absenkung auf einen pH-Wert zwischen 1,5 und 6 unterzogen wird.

6. Verfahren nach einem der Ansprüche 1-5, wobei die Mischung aus Ampicillin und 6-APA aus der Reaktionsmischung einer bei einem pH-Wert zwischen 7,5 und 10 durchgeführten enzymatischen Acylierungsreaktion durch Absenken des pH-Wertes auf einen Wert zwischen 7 und 9 und Isolieren der festen Substanz hergestellt wird.

7. Verfahren nach Anspruch 6, wobei zuerst die vorhandene feste Substanz aus der nach der Acylierungsreaktion erhaltenen Reaktionsmischung isoliert wird.

8. Verfahren nach einem der Ansprüche 1-5, wobei die Mischung aus Ampicillin und 6-APA aus der Reaktionsmischung einer enzymatischen Acylierungsreaktion bei einem pH-Wert zwischen 7 und 8,8 hergestellt wird, wobei die vorhandene feste Substanz isoliert wird.

## Revendications

1. Procédé pour la récupération de l'ampicilline à partir d'un mélange contenant de l'ampicilline et de l'acide 6-aminopénicillique (6-APA), **caractérisé en ce qu'**un mélange contenant de l'ampicilline et du 6-APA, ayant un pH supérieur à 7, homogène, en exceptant la présence d'ampicilline solide, à un pH entre 7 et 8,5, est soumis à un abaissement de pH jusqu'à atteinte d'un pH inférieur à 8,2, et **en ce que** la substance solide présente est récupérée.

2. Procédé selon la revendication 1, **caractérisé en ce que** le pH est diminué jusqu'à une valeur entre 5,5 et 7,8.

3. Procédé selon la revendication 1 ou 2, **caractérisé en ce que** le mélange de départ contient 2 à 60 % en mole de 6-APA, calculé par rapport à la quantité totale de 6-APA et d'ampicilline.

4. Procédé selon l'une quelconque des revendications 1 à 3, **caractérisé en ce que** la phase liquide résiduelle est soumise à une diminution du pH jusqu'à un pH inférieur à 7 et **en ce que** la substance solide présente est récupérée.

5. Procédé selon la revendication 4, **caractérisé en ce que** la phase liquide résiduelle est soumise à une diminution du pH jusqu'à un pH entre 1,5 et 6.

6. Procédé selon l'une quelconque des revendications 1 à 5, dans lequel le mélange d'ampicilline et de 6-APA est préparé à partir du mélange réactionnel d'une réaction d'acylation enzymatique réalisée à un pH entre 7,5 et 10, par abaissement du pH jusqu'à une valeur entre 7 et 9 et isolement de la substance solide.

7. Procédé selon la revendication 6, dans lequel, d'abord, la substance solide présente est isolée du mélange réactionnel obtenu après la réaction d'acylation.

8. Procédé selon l'une quelconque des revendications 1 à 5, dans lequel le mélange d'ampicilline et de 6-APA est préparé à partir du mélange réactionnel d'une réaction d'acylation enzymatique à un pH entre 7 et 8,8, avec isolement de la substance solide présente.
